# EUROPEAN PATENT APPLICATION

(11) **EP 4 578 858 A1**
(43) Date of publication of application: **02.07.2025**
(21) Application number: 23856716.8
(22) Date of filing: 25.08.2023
(51) Int. Cl.: C07D 487/04, C12P 17/18, C12P 41/00, A61P 35/00

(54) **PREPARATION METHOD FOR R-TYPE KETOROLAC AND USE THEREOF**

(30) Priority: 26.08.2022 CN 202211032611
(71) Applicant: YINUOKE MEDICINE SCIENCE AND TECHNOLOGY COMPANY LTD., Changchun, Jilin 130000 (CN)
(72) Inventor: ZHANG, Lingbing, Changchun, Jilin 130000 (CN); ZHANG, Dongxu, Changchun, Jilin 130000 (CN)
(74) Representative: J A Kemp LLP
(86) International application number: PCT/CN2023/114864
(87) International publication number: WO 2024/041629

(57) **Abstract**

Provided are a method for preparing (R)-ketorolac and use thereof, belonging to the technical field of organic synthesis. The method includes the following steps: step 1, preparing ketorolac; and step 2, subjecting the ketorolac to resolution to obtain the (R)-ketorolac, the resolution being selected from the group consisting of chiral amine resolution and enzyme resolution. In the disclosure, glacial acetic acid is used as a reaction solvent, which is not only clean and environmental-friendly, but also easy to be removed. The glacial acetic acid could significantly improve a conversion rate of SM1 as a raw material, thereby improving the yield and the purity of the ketorolac, such that the ketorolac has a yield of not less than 70% and a purity of not less than 99%. The ketorolac is subjected to chiral resolution by the chiral amine or the enzyme to obtain the (R)-ketorolac with a high purity and a high yield, and the (R)-ketorolac could prevent chemotherapy resistance and improve both chemotherapeutic efficacy and cure rate.

## Description

The present application claims priority to Chinese Patent Application No. CN202211032611.5 entitled "METHOD FOR PREPARING (R)-KETOROLAC AND USE THEREOF", filed with the China National Intellectual Property Administration (CNIPA) on August 26, 2022, which is incorporated herein by reference in its entirety as part of the present application.

### TECHNICAL FIELD

The present disclosure belongs to the technical field of organic synthesis, and specifically relates to a method for preparing (R)-ketorolac and use of the (R)-ketorolac.

### BACKGROUND

In recent years, people have realized that a series of activities of drugs *in vivo* are closely related to their structures, and different enantiomers show great differences in pharmacological activities, metabolic processes, and pharmacokinetics. Therefore, the study on synthesis and preparation of single enantiomers has become a hot topic in academic community at home and abroad. In nature, most of the amino acids in medicine, pesticides, and food industry are chiral substances, and generally exist as racemates of two or more enantiomers at the same time, which are several completely different substances, in terms of biological activity. Generally speaking, only one of the enantiomers shows a required biological activity, while the other one (ones) is redundant and affects an activity of the effective substance, or even cause opposite side effects. According to statistics, except for some natural drugs such as hormones and antibiotics that mainly exist in a single isomer, most other synthetic drugs are supplied as racemates among drugs currently on the market, thus obviously bringing some serious problems to the treatment of diseases and other applications. Accordingly, how to conduct chiral separation to obtain a single chiral drug and thereby control a quality of drug production has become a major issue of concern.

Ketorolac (also known as 5-benzoyl-1,2-dihydro-3H-pyrrolo[1,2-a]pyrrole-1-carboxylic acid), a new type of nonsteroidal anti-inflammatory analgesic, is potent, non-addictive, and non-narcotic and produces a strong analgesic effect by inhibiting the synthesis of prostaglandins. The racemic ketorolac has been on the market in the United States since 1991 and is sold as ketorolac tromethamine.

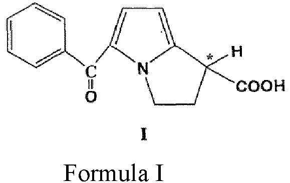

Ketorolac molecule has a structure as shown in Formula I, in which there is a chiral carbon atom, and thus has a pair of enantiomers. Studies have shown that (S)-ketorolac exhibits an analgesic effect 230 times and an anti-inflammatory effect 60 times stronger than those of (R)-ketorolac. In view of this, people have been constantly seeking ways to obtain the (S)-ketorolac since the ketorolac was on the market.

Chinese patent application No. CN02102547.9 discloses a method for preparing optically active ketorolac. In the method, two amides generated by the kinetic resolution of the first step do not appear simultaneously, but appear one after another. The most ideal situation is that one isomer of racemic ketorolac is completely converted into an amide product before the other isomer begins to react. For the kinetic resolution, in principle, as long as the reaction is terminated before the racemic raw material is completely converted into the product, both the reaction product and the unreacted raw material should be optically active. That is to say, the reaction can show selectivity and achieve resolution. What is finally resolved by the method of the application is an (S)-isomer.

However, there are few reports on the preparation of (R)-isomers. Chinese patent application No. CN202111115983.X discloses use of (R)-ketorolac in prevention and treatment of aortic dissection and aortic aneurysm. Oral administration of the (R)-ketorolac could inhibit pathological dilation of the aorta and reduce the incidence and mortality of aortic dissection and aortic aneurysm. Moreover, the (R)-ketorolac can inhibit the occurrence of inflammatory reactions in the blood vessel wall, reduce the rupture of aortic aneurysm, inhibit the occurrence of intraluminal hematoma, and maintain the integrity of elastic fibers on the blood vessel wall, thereby treating the aortic dissection and aortic aneurysm. Use in this patent application opens up a new application field of the (R)-ketorolac, and provides a meaningful reference for preventing and treating aortic dissection and aortic aneurysm to improve vascular lesions. However, this application only discloses use of the (R)-ketorolac in prevention and treatment of the aortic dissection and aortic aneurysm, and does not involve the preparation of (R)-isomers.

Chinese patent application No. CN201880084997.X discloses use of (R)-ketorolac in treatment of cachexia and provides a method for treating the cachexia to reduce morbidity and mortality from chronic diseases and generally improve an effectiveness of treating the chronic diseases. However, this application does not involve the preparation of (R)-ketorolac either.

Accordingly, there is a need to provide a method for preparing (R)-ketorolac.

### SUMMARY

In view of the deficiencies in the prior art, the present disclosure aims to provide a method for preparing (R)-ketorolac and use of the (R)-ketorolac. The present disclosure enables the (R)-ketorolac to have a higher purity by optimizing the preparation method. Moreover, the (R)-ketorolac is able to combine with chemotherapy to significantly increase a curative effect of the chemotherapy, thereby improving a cure rate of the tumors.

To solve the foregoing technical problems, the present disclosure provides the following technical solutions:
The present disclosure provides a method for preparing (R)-ketorolac, including the following steps:
step 1, preparing ketorolac:
   (1) adding glacial acetic acid, manganese acetate dihydrate, triethyl methanetricarboxylate (SM2), 2-benzoylpyrrole (SM1), and sodium acetate into a fourth reactor, heating and reacting completely to obtain a reaction system, cooling the reaction system, adding MTBE thereto, and filtering a resulting system to obtain a filtrate; adjusting the filtrate to have a pH value of 4 to 8 by adding a potassium carbonate aqueous solution, then subjecting the filtrate to liquid separation to collect an organic phase, subjecting the organic phase to concentration, recrystallization, filtration, and drying in sequence to obtain product A, i.e., triethyl (5-benzoyl-1H-pyrrol-2-yl)methanetricarboxylate;
      where step (1) is conducted according to the following reaction equation: 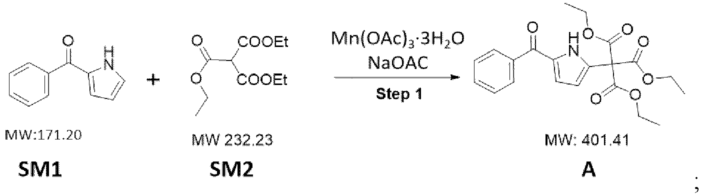 and
   (2) adding 1,2-dichloroethane, the product A, potassium carbonate, and tetrabutylammonium bromide (TBAB) into a fifth reactor, and subjecting a resulting system to first heating, and reacting completely to obtain a mixed solution containing product B, i.e., diethyl 5-benzoyl-2,3-dihydro-1H-pyrrolizine-1,1-dicarboxylate; filtering the mixed solution containing the product B to obtain a filtrate, introducing the filtrate into a sixth reactor and conducting concentration, then adding a tetrahydrofuran (THF) solution and a sodium hydroxide solution into the sixth reactor in sequence, subjecting a resulting system to second heating, and reacting completely to obtain a product, subjecting the product to liquid separation to obtain an organic phase, collecting the organic phase, and adjusting the organic phase to have a pH value of 3 by adding a solution of HCl in THF to obtain a mixture solution, subjecting the mixture solution to concentration to obtain a concentrate, adding MTBE into the concentrate, and washing with water, subjecting a resulting washed product to liquid separation to obtain an organic phase, collecting the organic phase, subjecting the organic phase to decolorization, recrystallization filtration, and drying to obtain product C, i.e., the ketorolac;
      where step (2) is conducted according to the following reaction equation: 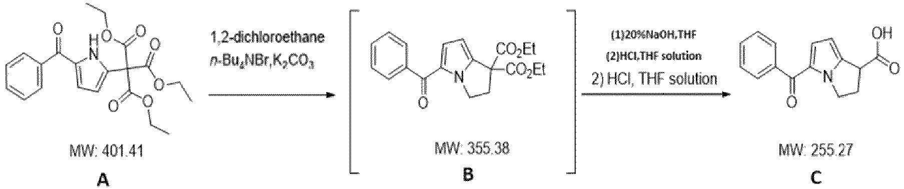
step 2, subjecting the ketorolac to resolution to obtain the (R)-ketorolac, the resolution being selected from the group consisting of chiral amine resolution and enzyme resolution;
where the chiral amine resolution includes steps of:
   adding the ketorolac and isopropyl alcohol into a first reactor, heating to dissolve the ketorolac, adding a chiral amine and a seed crystal thereto, stirring to be uniform, adding ethyl acetate dropwise thereto, subjecting a resulting mixture to cooling crystallization and filtration in sequence to obtain solid 1 and a filtrate, acidifying the solid 1 with hydrochloric acid to obtain an acidified product, and filtering the acidified product to obtain product FP, i.e., the (R)-ketorolac;
   where the chiral amine resolution is conducted according to the following reaction equation: 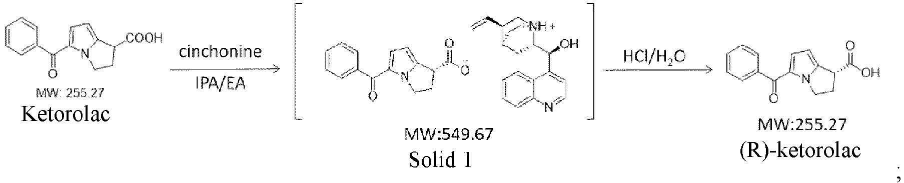 and
   the enzyme resolution includes steps of:
      (i) adding the ketorolac and methanol into a second reactor, cooling to a temperature of -5°C to 5°C, adding SOCl₂ thereto, heating a resulting system to a temperature of 40°C to 50°C and reacting completely to obtain a reactant, subjecting the reactant to concentration, then crystallization by adding water, filtration, and drying in sequence to obtain product D, i.e., ketorolac methyl ester;
         where step (i) is conducted according to the following reaction equation: 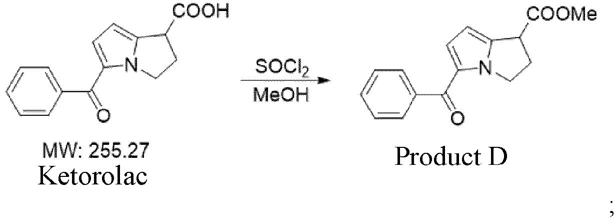 and
      (ii) adding the product D, tert-butyl alcohol (TBA), a buffer solution, and an enzyme into a third reactor in sequence, reacting while monitoring a chiral purity; after the reacting is completed, filtering a resulting reaction product to obtain a filtrate, washing by adding methyl tert-butyl ether (MTBE) and water, and subjecting a resulting washed system to liquid separation to obtain an organic phase, subjecting the organic phase to concentration and then further liquid separation to obtain a further organic phase, collecting the further organic phase and adding MTBE, cooling to a temperature of -5°C to 5°C, adding isopropylamine thereto and stirring, filtering a resulting mixture system to obtain a solid; adding the solid into the reactor and then acidifying the solid with water and 4 mol/L hydrochloric acid aqueous solution to obtain an acidified product, and filtering the acidified product to obtain product FP, i.e., the (R)-ketorolac;
         where step (ii) is conducted according to the following reaction equation:

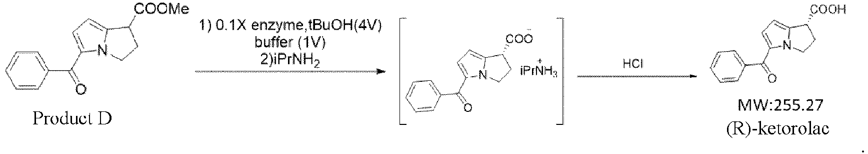

In some embodiments, in step (1), a molar ratio of the 2-benzoylpyrrole, the triethyl methanetricarboxylate, the manganese acetate dihydrate, the sodium acetate, and the glacial acetic acid is in a range of 0.99-1.01 : 1.09-1.12 : 1.9-2.1 : 2.8-3.2 : 28-32.

In further embodiments, in step (1), a molar ratio of the 2-benzoylpyrrole, the triethyl methanetricarboxylate, the manganese acetate dihydrate, the sodium acetate, and the glacial acetic acid is 1:1.1:3:2:30.

In some embodiments, in step (1), a mass ratio of the MTBE to the 2-benzoylpyrrole is 18:1.

In some embodiments, in step (1), heating and reacting is conducted at a temperature of 65°C to 75°C, preferably 70°C; and heating and reacting is conducted for 14 h to 20 h, preferably 16 h.

In some embodiments, in step (1) the cooling is conducted at a temperature of 35°C to 45°C, preferably 40°C.

In some embodiments, in step (1), the recrystallization is conducted by adding ethanol at a mass ratio of the ethanol to the 2-benzoylpyrrole being 4:1 into a concentrated organic phase to obtain a system, heating the system to a temperature of 40°C and dissolving a resulting solid while stirring to obtain a solution, cooling the solution to a temperature of -10°C so that the resulting solid is crystallized, and subjecting a resulting product to filtration and vacuum drying in sequence to obtain product A, wherein the cooling is conducted for 6 h to 10 h.

In some embodiments, in step (1), reacting completely (complete reaction) is determined based on a standard of SM1/A≤5% (i.e., in a high-performance liquid chromatography (HPLC) spectrum, a ratio of a peak area of SM1 to that of the product A is not greater than 5%).

In some embodiments, in step (2), a molar ratio of the 1,2-dichloroethane, the product A, the potassium carbonate, and the TBAB is in a range of 85-95 : 0.99-1.01 : 8.7-11.6 : 0.75-1.25.

In further embodiments, in step (2), a molar ratio of the 1,2-dichloroethane, the product A, the potassium carbonate, and the TBAB is 90:1:10:1.

In some embodiments, in step (2), the first heating is conducted at a temperature of 75°C to 85°C, preferably 80°C; the reacting is conducted for 14 h to 20 h, preferably 18 h; and the reacting completely is determined based on a standard of product A/product B≤5% (i.e., in HPLC spectrum, a ratio of a peak area of the product A to that of the product B is not greater than 5%).

In some embodiments, in step (2), the THF solution is added in an amount of a mass ratio of the THF solution to the product A being 8:1.

In some embodiments, in step (2), the sodium hydroxide solution has a mass percentage of 20%, and the sodium hydroxide solution is added in an amount of a mass ratio of the sodium hydroxide solution to the product A being 4:1.

In some embodiments, in step (2), the second heating is conducted at a temperature of 50°C to 60°C, preferably 55°C, the reacting is conducted for 1 h to 5 h, preferably 5 h, and the reacting completely is determined based on a standard of B/C≤5% (i.e., in HPLC spectrum, a ratio of a peak area of the product B to that of the product A is not greater than 5%).

In some embodiments, in step (2), the solution of HCl in THF has a mass percentage of 11%, and an addition amount of a mass ratio of the solution of HCl in THF to the product A being 1.45:1.

In some embodiments, in step (2), a mass ratio of the MTBE to the product A is 7:1.

In some embodiments, in step (2), the decolorization is conducted by using a CUNO filter. and

In some embodiments, in step (2), the recrystallization is performed by: concentrating a decolorized organic phase to obtain a concentrated organic phase, adding n-heptane into the concentrated organic phase to obtain a mixture, heating the mixture to a temperature of 40°C and dissolving a resulting solid while stirring to obtain a mixed solution, slowly cooling the mixed solution to a temperature of -10°C so that the resulting solid is crystallized, and subjecting a resulting product to filtration and vacuum drying in sequence to obtain the product C.

In some embodiments, in the chiral amine resolution, the isopropyl alcohol is added in an amount of a mass ratio of the isopropyl alcohol to the product C (ketorolac) being 4.2:1 to 8.0:1, preferably 4.75:1.

In some embodiments, in the chiral amine resolution, the heating is conducted at a temperature of 50°C to 60°C, preferably 55°C.

In some embodiments, in the chiral amine resolution, the chiral amine is one selected from the group consisting of dehydroabietylamine (DHAA), (s)-1-phenylethylamine, (1S,2S)-(+)-1,2-diaminocyclohexane, L-(-)-epinephrine, (R)-(+)-1-(1-naphthyl)ethylamine, and cinchonine; and in further embodiments, the chiral amine is the cinchonine.

In some embodiments, a molar ratio of the product C (ketorolac) to the chiral amine is in a range of 1:0.82 to 1:1.73.

In some embodiments, a molar ratio of the product C (ketorolac) to the seed crystal is in a range of 1:0.0005 to 1:0.05.

In some embodiments, in the chiral amine resolution, the chiral amine is added by batch addition; and the batch addition includes steps of: firstly adding the chiral amine at a molar ratio of the chiral amine to the product C being 0.52:1 to 0.86:1, and dissolving solid by stirring, secondly adding the chiral amine at a molar ratio of the chiral amine to the product C being 0.04:1 to 0.17:1, and dissolving the solid by stirring, then thirdly adding the chiral amine at a molar ratio of the chiral amine to the product C being 0.04:1 to 0.17:1, stirring for 1 h, fourthly adding the chiral amine at a molar ratio of the chiral amine to the product C being 0.04:1 to 0.17:1, stirring for 1.5 h, and finally adding the chiral amine at a molar ratio of the chiral amine to the product C being 0.17:1 to 0.35:1.

In some embodiments, the chiral amine is added by: firstly adding the cinchonine at a molar ratio of the cinchonine to the product C being 0.68:1, dissolving solid by stirring; secondly adding the cinchonine at a molar ratio of the cinchonine to the product C being 0.07:1, dissolving the solid by stirring; thirdly adding the cinchonine at a molar ratio of the cinchonine to the product C being 0.13:1, stirring for 1 h, fourthly adding the cinchonine at a molar ratio of the cinchonine to the product C being 0.1:1, stirring for 1.5 h, and finally adding the cinchonine at a molar ratio into the cinchonine to the product C being 0.25:1.

In some embodiments, the seed crystal is added after secondly adding the chiral amine.

In some embodiments, in the chiral amine resolution, the ethyl acetate is added in an amount of a mass ratio of the ethyl acetate to the ketorolac being 5: 1, and the method further includes: after adding the ethyl acetate dropwise is completed, stirring at a temperature of 50°C to 60°C for 6 h to 12 h; in some embodiments, the stirring is conducted at 55°C for 9 h.

In some embodiments, in the chiral amine resolution the cooling is conducted by cooling to 15°C.

In some embodiments, the chiral amine resolution further includes: concentrating the filtrate and then added the ethyl acetate thereto, stirring, and then subjecting a resulting mixture to cooling crystallization to obtain a crystallized system, filtering the crystallizing system to obtain solid 2 and further filtrate, the further filtrate being discarded, combining the solid 1 and the solid 2, acidifying a resulting solid mixture by adding hydrochloric acid, and filtering a resulting acidified system to obtain the product FP, i.e., the (R)-ketorolac.

In some embodiments, the solid 1 and the solid 2 are both cinchonine salts of the (R)-ketorolac; the solid 1 is the cinchonine salt of the (R)-ketorolac obtained by first salt formation; the solid 2 is the cinchonine salt of the (R)-ketorolac obtained by recrystallization of a filtrated mother liquor after the first salt formation.

In some embodiments, the ethyl acetate is added in an amount of a mass ration of the ethyl acetate to the ketorolac being 8: 1, and the cooling is conducted at a temperature of 20°C.

In some embodiments, in the enzyme resolution, the enzyme is Novozym 435, and a mass ratio of the enzyme to the product D is 10: 1.

In some embodiments, in the enzyme resolution, the buffer solution is a potassium phosphate buffer solution with a pH of 7.0.

The present disclosure further provides use of (R)-ketorolac prepared by the method as described in the above solutions in preparation of a drug for treating a cancer. In some embodiments, the cancer is one selected from the group of consisting of colon cancer and breast cancer.

Compared with the prior art, the present disclosure has the following beneficial effects:
(1) In the prior art, high-boiling toluene as a solvent is difficult to be removed and inconvenient for crystallization. In the present disclosure, glacial acetic acid is used as a reaction solvent, which is not only clean and environmental-friendly, but also easy to be removed. The glacial acetic acid could significantly improve a conversion rate of SM1 as a raw material, thereby improving the yield and the purity of the ketorolac, such that the ketorolac has a yield of not less than 70% and a purity of not less than 99%.
(2) In the present disclosure, the ketorolac is subjected to chiral resolution by using chiral amine or enzyme to obtain (R)-ketorolac with high purity and high yield, and the (R)-ketorolac could prevent chemotherapy resistance and improve both chemotherapeutic efficacy and cure rate.

### BRIEF DESCRIPTION OF THE DRAWINGS

FIG. 1A and FIG. 1B show a liquid chromatogram and relevant data of the product A prepared in Example 1;
FIG. 2A and FIG. 2B show a liquid chromatogram and relevant data of the product C prepared in Example 1;
FIG. 3A and FIG. 3B show a liquid chromatogram and relevant data of the product FP prepared by the chiral amine resolution in Example 1;
FIG. 4A and FIG. 4B show a high-performance liquid chromatography (HPLC) spectrum and relevant data of the chiral purity of the product FP prepared by the chiral amine resolution in Example 1;
FIG. 5A and FIG. 5B show a liquid chromatogram and relevant data of the product D prepared in Example 1;
FIG. 6A and FIG. 6B show a liquid chromatogram and relevant data of the product FP prepared by the enzyme resolution in Example 1;
FIG. 7A and FIG. 7B show an HPLC spectrum and relevant data of the chiral purity of the product FP prepared by the enzyme resolution in Example 1;
FIG. 8A and FIG 8B show a liquid chromatogram and relevant data of the product A prepared in Example 2;
FIG. 9A and FIG 9B show a liquid chromatogram and relevant data of the product C prepared in Example 2;
FIG. 10A and FIG. 10B show a liquid chromatogram and relevant data of the product FP prepared by the chiral amine resolution in Example 2;
FIG. 11A and FIG. 11B show an HPLC spectrum and relevant data of the chiral purity of the product FP prepared by the chiral amine resolution in Example 2;
FIG. 12A and FIG. 12B show a liquid chromatogram and relevant data of the product A prepared in Example 3;
FIG. 13A and FIG. 13B show a liquid chromatogram and relevant data of the product C prepared in Example 3;
FIG. 14A and FIG. 14B show a liquid chromatogram and relevant data of the product FP prepared by the chiral amine resolution in Example 3;
FIG. 15A and FIG. 15B show an HPLC spectrum and relevant data of the chiral purity of the product FP prepared by the chiral amine resolution in Example 3;
FIG. 16 shows a tumor growth curve of the (R)-ketorolac combined with gemcitabine in the colon cancer model Colon-26;
FIG. 17 shows a tumor growth curve of the (R)-ketorolac combined with cyclophosphamide in the colon cancer model Colon-26; and
FIG. 18 shows a tumor growth curve of the (R)-ketorolac combined with cyclophosphamide in the breast cancer model 4T1.

### DETAILED DESCRIPTION OF THE EMBODIMENTS

The technical solutions in the present disclosure will be further described below in conjunction with specific examples, but the scope claimed is not limited to these descriptions.

In the present disclosure, the purchasing manufacturers and models of the raw materials used herein are as follows:

| Raw material | Manufacturer (merchant) | Model (batch number) |
|---|---|---|
| 2-benzoylpyrrole (SM1) | Shanghai Renshi Pharmatech Co., Ltd., China | Batch number: RSP0322002 |
| Triethyl methanetricarboxylate (SM2) | Chengdu Xiaojia Technology Co., Ltd., China | Batch number: J2073-99 |
| Glacial acetic acid | Shanghai Huayi Energy Chemical Co., Ltd., China | Batch number: 2021-07-15-1 |
| Manganese acetate | Beijing Donghua Lituo Technology and Development Co., Ltd. | Batch number: 200905 |
| 1,2-dichloroethane | Shanghai Chlor-Alkali Chemical Co., Ltd., China | Batch number: 20210320 |
| Potassium carbonate | Shanghai Jiarong Trading Co., Ltd. | Batch number: M210155 |
| Sodium hydroxide | Shanghai Chlor-Alkali Chemical Co., Ltd., China | Batch number: 20210603 |
| Isopropanol | Kellin Chemicals Co., Ltd., China | Batch number: N210531T |
| Cinchonine | BUCHLER | Batch number: 9868 |
| THF | BASF | Batch number: 01210607T |
| Ethyl acetate | Anhui Huayi Chemical Co., Ltd., China | Batch number: 2021-04-16-1 |
| Anhydrous sodium acetate | Wuxi Yangshan Biochemical Co., Ltd., China | Batch number: 01200510 |

### Example 1: Preparation method of (R)-ketorolac

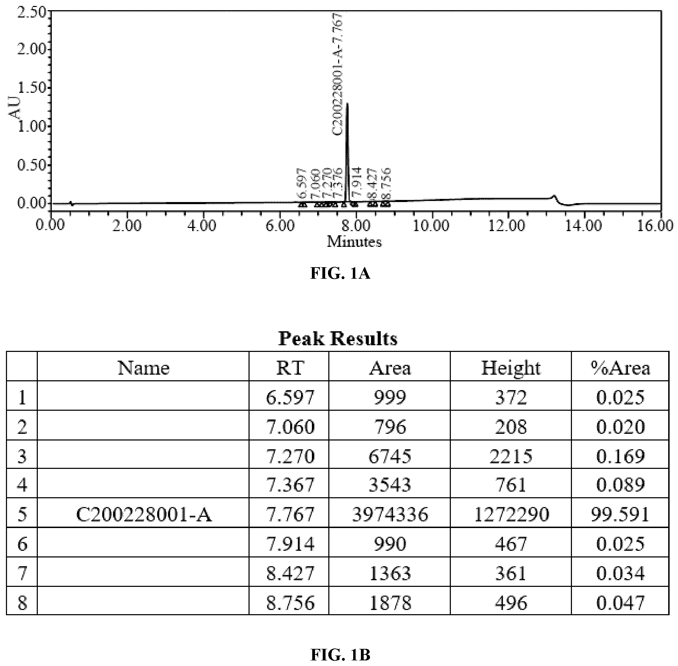

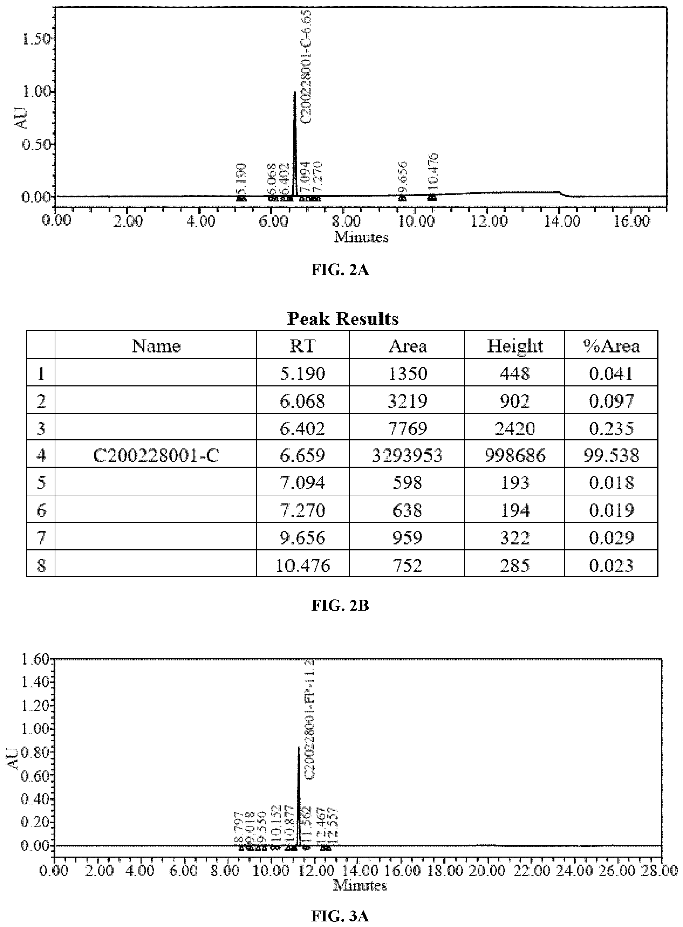

The (R)-ketorolac was prepared as follows:
Step 1, preparation of ketorolac:
   (1) Glacial acetic acid (11,015 g), manganese acetate dihydrate (trivalent, 4,087 g), triethyl methanetricarboxylate (SM2, 1,300 g), 2-benzoylpyrrole (SM1, 870 g), and sodium acetate (835 g) were added into a reactor, heated to 65°C to 75°C and reacted completely. A resulting reaction system was cooled to a temperature of 35°C to 45°C, and MTBE (7,000 mL) was then added thereto. A resulting mixture was filtered to obtain a filtrate, and the filtrate was adjusted to have a pH of 4 to 8 by adding a potassium carbonate aqueous solution into the filtrate. The resulting filtrate was subjected to liquid separation to collect an organic phase, and the organic phase was subjected to concentration, recrystallization, filtration and drying to obtain product A (1,220 g, with a yield of 59.80% and a purity of 99.59% (see FIGs. 1A and 1B)), where
      step (1) was conducted according to the following reaction equation: 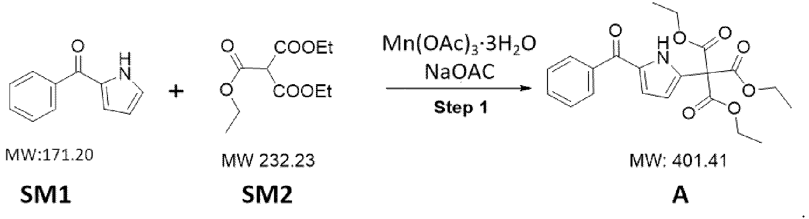
   (2) 1,2-dichloroethane (28,767 g), the product A (1,144 g), potassium carbonate (3,939 g), and TBAB (918.74 g) were added into a reactor, and heated to a temperature of 75°C to 85°C and reacted completely to obtain a mixed solution containing product B. The mixed solution containing the product B was filtered, a resulting filtrate was transferred into the reactor and subjected to concentration therein. After that, a THF solution (6,800 mL) and a sodium hydroxide solution (20%, 4.0 kg) were added into the reactor in sequence, and a resulting system was heated to 55°C and reacted completely. A resulting product was subjected to liquid separation, and a resulting organic phase was collected and adjusted to have a pH of 3 by adding a solution of HCl in THF thereto. A resulting mixture was concentrated to obtain a concentrate, and the MTBE (5,000 mL) was added into the concentrate. A resulting product was washed with water and then subjected to liquid separation, and a resulting organic phase was collected, and subjected to decolorization and recrystallization, filtration and drying in sequence to obtain product C (303 g, with a yield of 41.8% and a purity of 99.54% (see FIGs. 2A and 2B)), i.e., the ketorolac, where
step (2) was conducted according to the following reaction equation: 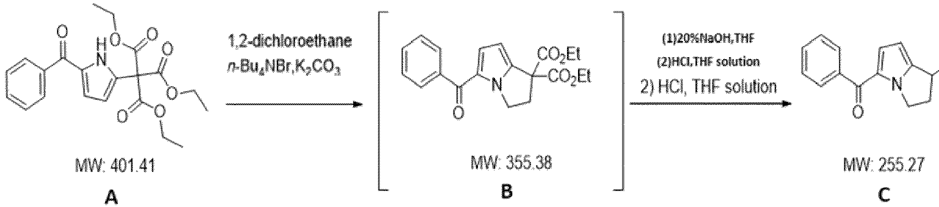
Step 2, Resolution of the (R)-ketorolac:

### 2.1 Chiral amine resolution:

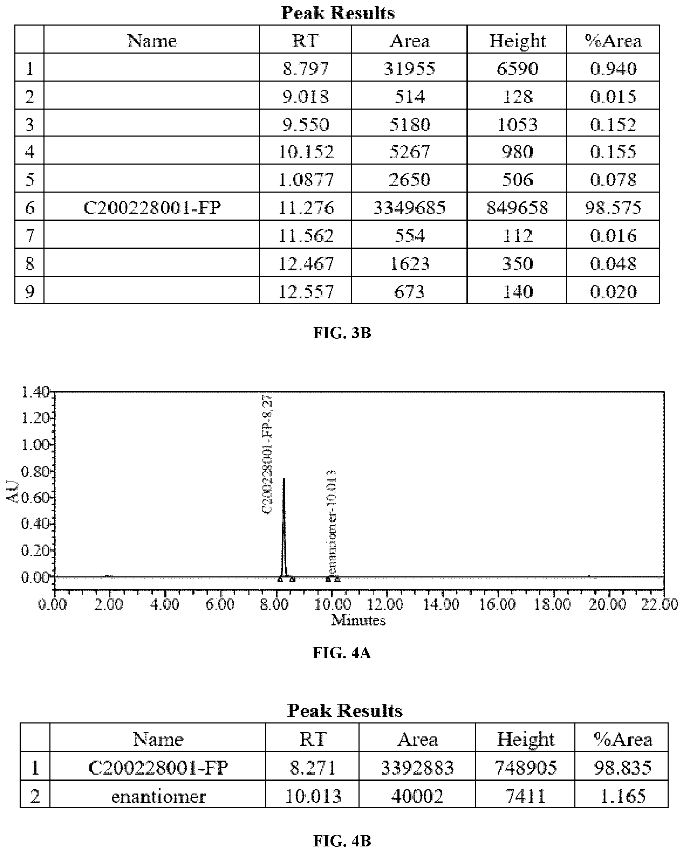

The product C (286 g) and isopropyl alcohol (1,610 mL) were added into a reactor, and heated at 55°C, such that the product C was dissolved to obtain a system. A chiral amine (386.76 g) and a seed crystal (4.0 g) were added into the system and stirred to be uniform, and ethyl acetate (1,610 mL) was then added dropwise. A resulting system was subjected to cooling crystallization, and then filtered to obtain solid 1 and a filtrate. The solid 1 was acidified with hydrochloric acid (4 mol/L, 1,275 mL), and a resulting acidified system was filtered to obtain product FP (211.98 g, with a yield of 74%, a purity of 98.58% (see FIGs. 3A and 3B) and a chiral purity of 98.84% (see FIGs. 4A and 4B)) i.e., the (R)-ketorolac; where
chiral amine resolution was conducted according to the following reaction equation:

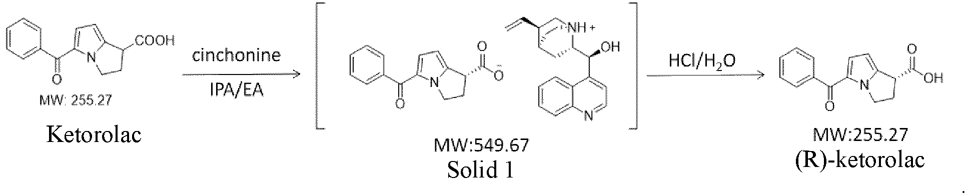

### 2.2 Enzyme resolution:

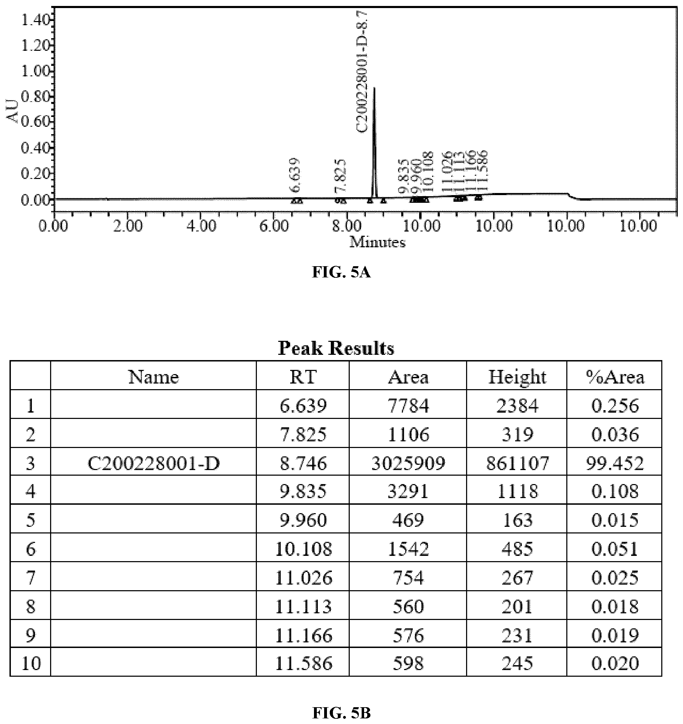

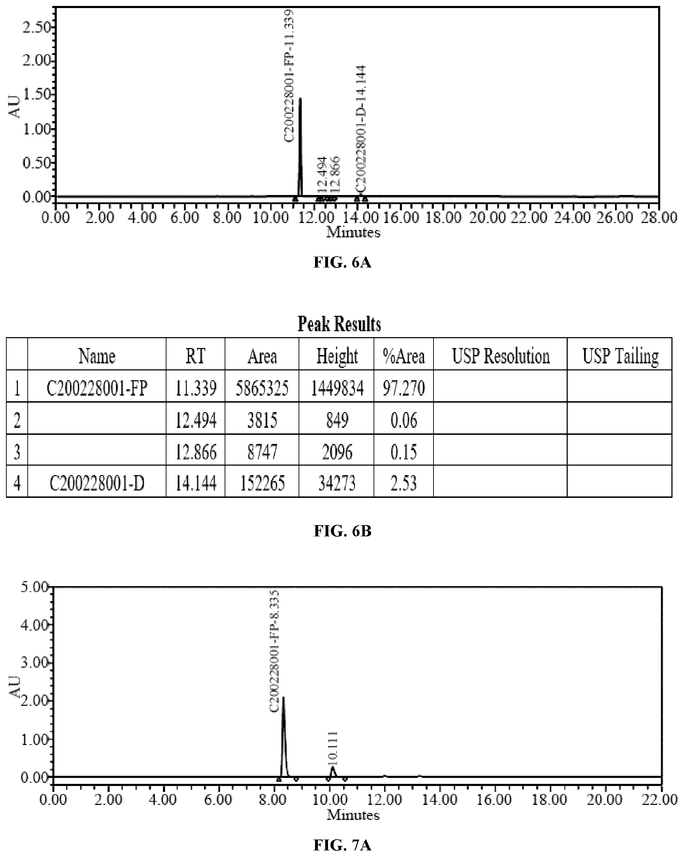

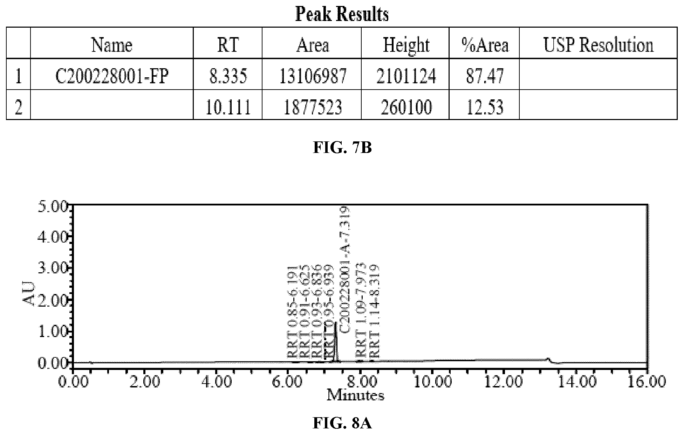

(i) The product C (180 g) and methanol (2,844 g) were added into a reactor, and cooled to a temperature of -5°C to 5°C, and SOCl₂ (126 g) was then added thereto to obtain a mixture. The mixture was heated to a temperature of 40°C to 50°C and reacted completely, and a resulting reactant was concentrated and subjected to crystallization by adding a process water (900 g), then filtration and drying to obtain product D (178.69 g, with a yield of 94% and a purity of 99.45% (FIGs. 5A and 5B)); where
   step (i) was conducted according to the following reaction equation: 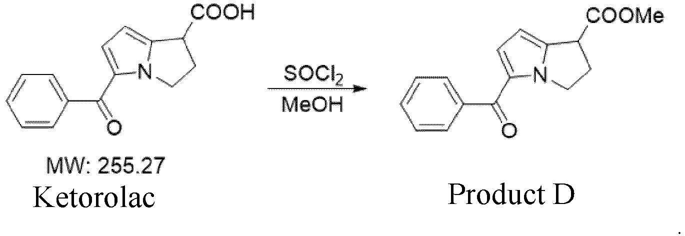
(ii) The product D (100 g), TBA (400 mL), a buffer solution (100 mL), and an enzyme (10 g) were added into a reactor in sequence, and a resulting mixture was adjusted to have a temperature of 25°C to 35°C and reacted completely. A resulting reactant was filtered, and a resulting filtrate was washed with MTBE (100 mL) and a process water (150 mL), and then subjected to liquid separation, and a resulting organic phase was concentrated and subjected to further liquid separation to obtain a further organic phase. The MTBE (150 mL) and isopropylamine (12.2 g) were added into the further organic phase to form a salt. A resulting system containing the salt was filtered to obtain a solid, and the solid was acidified with a hydrochloric acid aqueous solution (45 mL, 4 mol/L) to obtain product FP (35.91 g, with a yield of 39.6%, a purity of 97.27% (see FIGs. 6A and 6B), and a chiral purity of 87.47% (FIGs. 7A and 7B)); where
   step (ii) was conducted according to the following reaction equation:

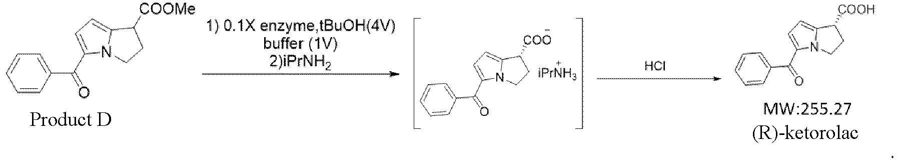

### Example 2: Preparation method of (R)-ketorolac

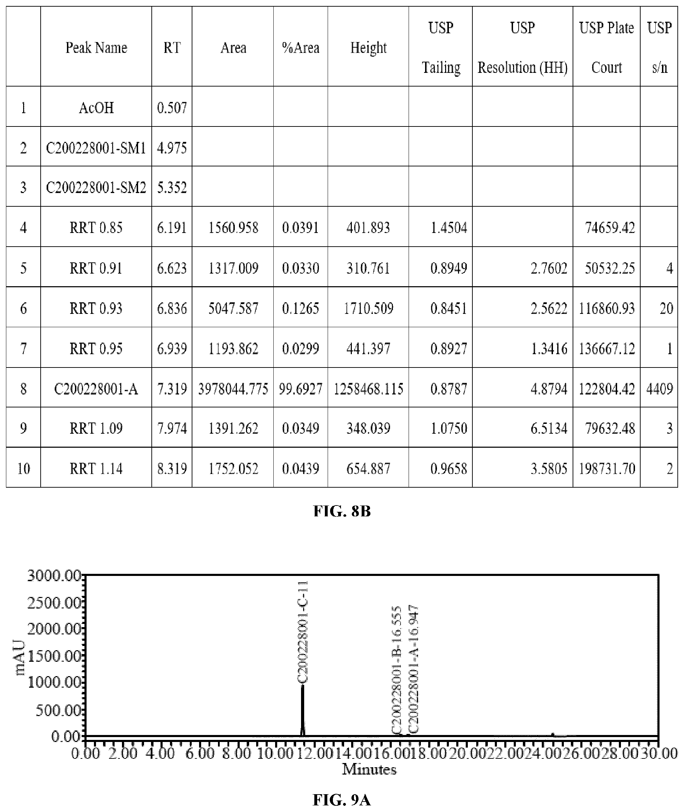

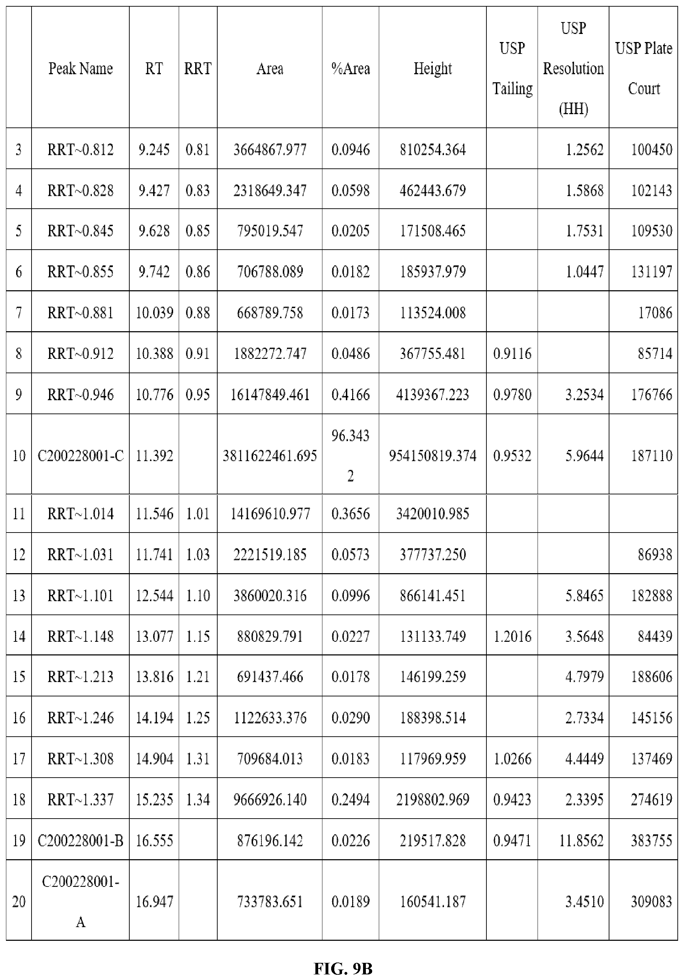

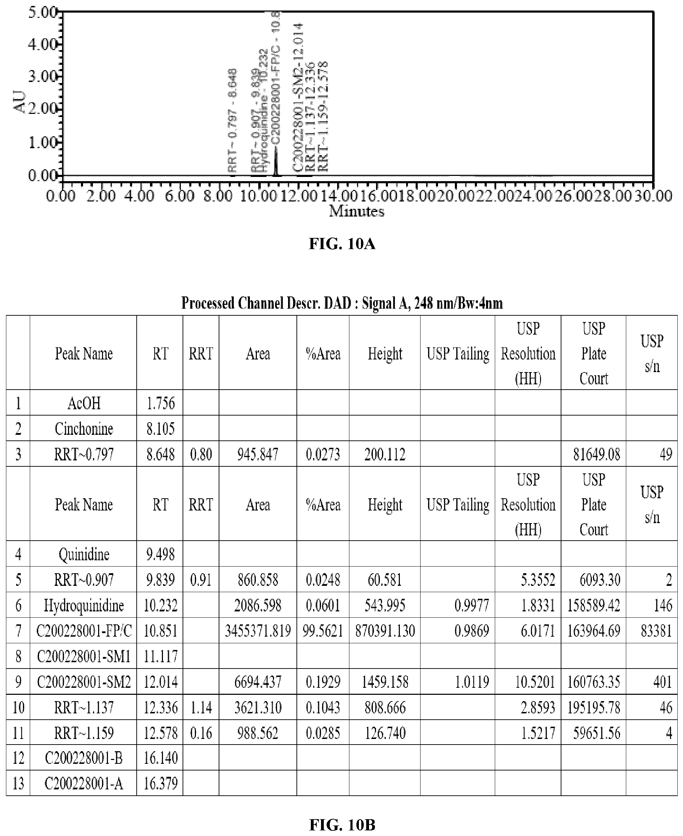

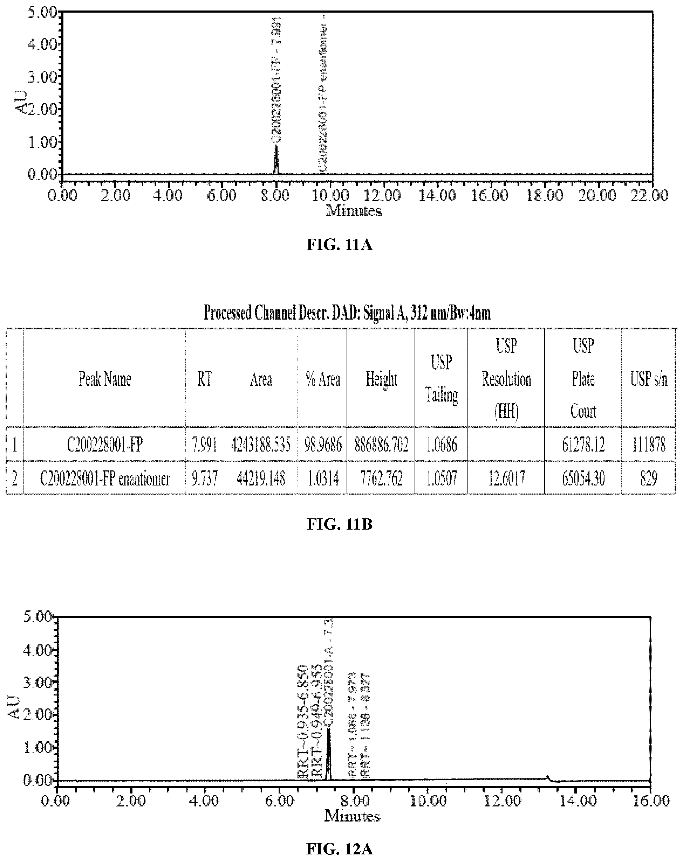

The (R)-ketorolac was prepared as follows:
Step 1, preparation of ketorolac:
   (1) Glacial acetic acid (18,980 g), manganese acetate dihydrate (trivalent, 4,700 g), triethyl methanetricarboxylate (SM2, 1,500 g), 2-benzoylpyrrole (SM1, 1,000 g), and sodium acetate (960 g) were added into a reactor, heated to 65°C to 75°C and reacted completely. A resulting reaction system was cooled to a temperature of 35°C to 45°C, and MTBE (7,000 mL) was added thereto. A resulting mixture was filtered to obtain a filtrate, and the filtrate was adjusted to a pH of 4 to 8 by adding a potassium carbonate aqueous solution into the filtrate. The resulting adjusted filtrate was subjected to liquid separation to collect an organic phase, and the organic phase was subjected to concentration, recrystallization, filtration and drying to obtain product A (1,740 g, with a yield of 74% and a purity of 99.69% (see FIGs. 8A and 8B)).
      Step (1) was conducted according to the following reaction equation: 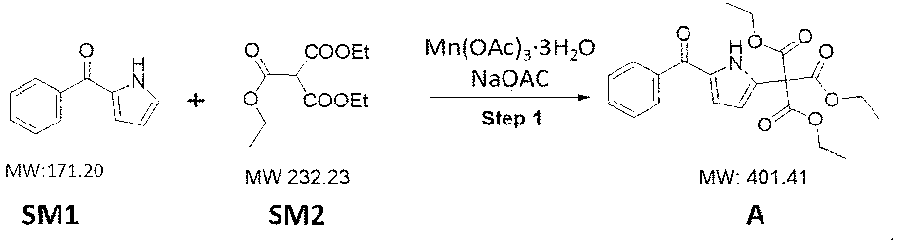
   (2) 1,2-dichloroethane (36.04 kg), the product A (1.72 kg), potassium carbonate (6.14 kg), and TBAB (1.4 kg) were added into a reactor, and heated to 80°C and reacted completely to obtain a mixed solution containing product B. The mixed solution containing the product B was filtered, a resulting filtrate was transferred to the reactor and subjected to concentration therein. After that, a THF solution (12.02 kg) and a sodium hydroxide solution (7.00 kg) were added into the reactor in sequence, and a resulting system was heated to 54°C and reacted completely. A resulting product was subjected to liquid separation, a resulting organic phase was collected, and adjusted to have a pH of 3 by adding a solution of HCl in THF (2.5 kg), A resulting mixture was concentrated to obtain a concentrate, and the MTBE (6.10 kg) was added into the concentrate. A resulting product was washed with water and then subjected to liquid separation, and a resulting organic phase was collected, and subjected to decolorization and recrystallization, filtration and drying in sequence to obtain product C (0.64 kg, with a yield of 56% and a purity of 98.34% (see FIGs. 9A and 9B)), i.e., the ketorolac; where
      step (2) was conducted according to the following reaction equation: 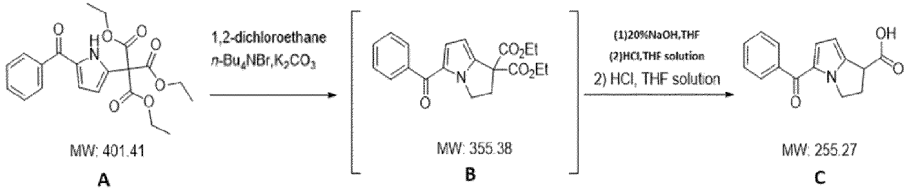
Step 2, Resolution of the (R)-ketorolac:
   The product C (0.56 kg) and isopropyl alcohol (2.50 kg) were added into a reactor, and heated at a temperature of 55°C, such that the product C was dissolved to obtain a system. A chiral amine (0.70 kg) and a seed crystal (0.011 kg) were added into the system, and stirred to be uniform, and ethyl acetate (2.88 kg) was then added thereto dropwise. A resulting system was subjected to cooling crystallization and then filtered to obtain solid 1 and a filtrate. The solid 1 was acidified with hydrochloric acid (4 mol/L, 2.10 kg), and a resulting acidified system was filtered to obtain product FP (0.32 kg, with a yield of 54%, a purity of 99.56% (see FIGs. 10A and 10B), and a chiral purity of 98.97% (see FIGs. 11A and 11B)), i.e., the (R)-ketorolac; where
   step 2 was conducted according to the following reaction equation:

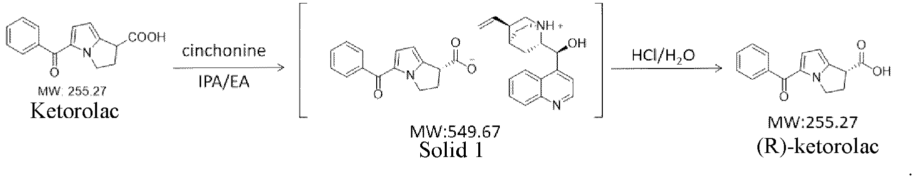

### Example 3: Preparation method of (R)-ketorolac

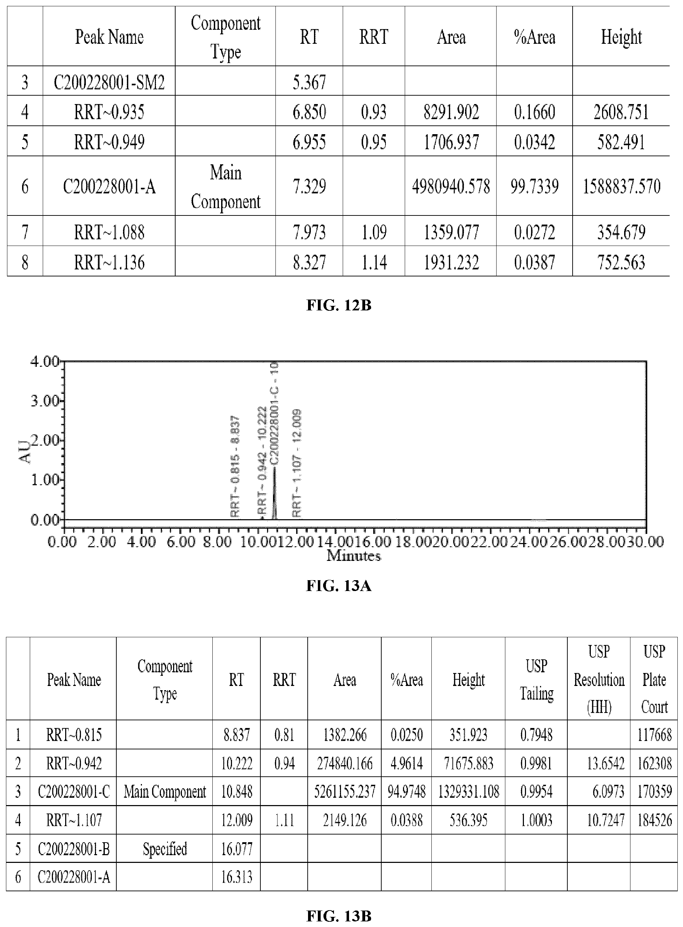

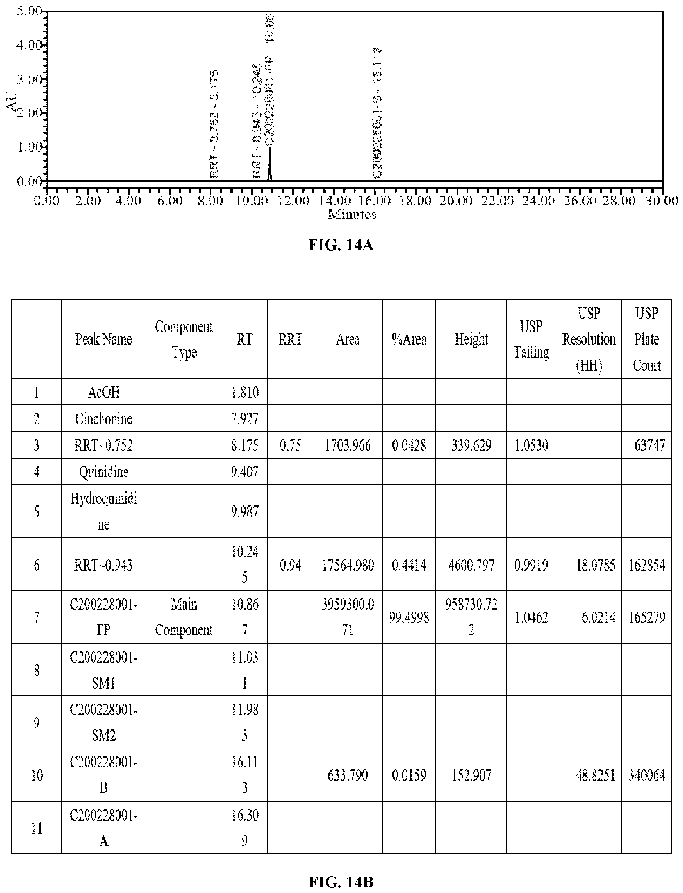

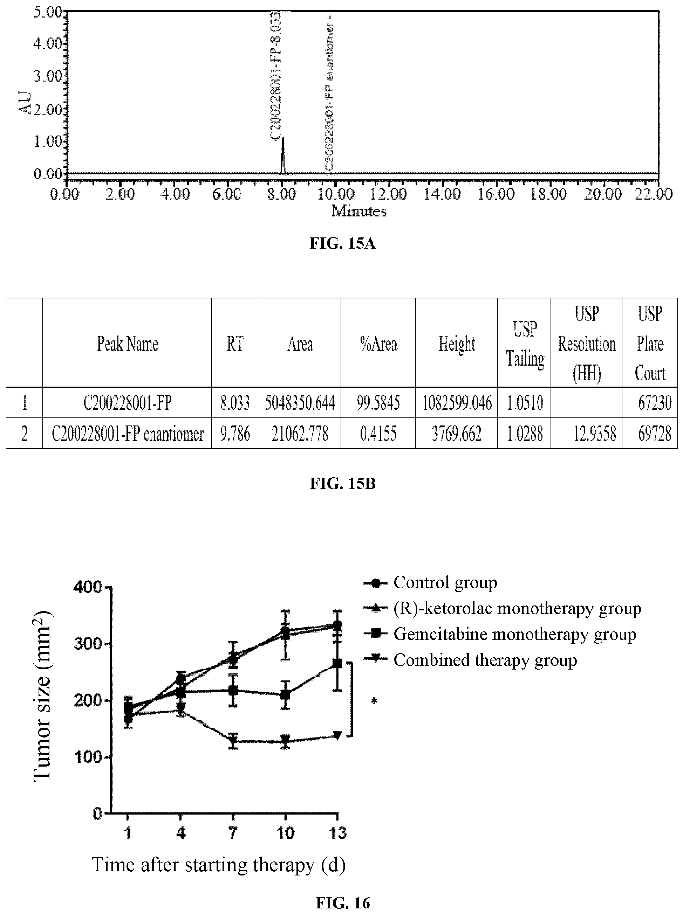

The (R)-ketorolac was prepared as follows.
Step 1, preparation of ketorolac:
   (1) Glacial acetic acid (28.74 kg), manganese acetate dihydrate (trivalent, 8.01 kg), triethyl methanetricarboxylate (SM2, 2.56 kg), 2-benzoylpyrrole (SM1, 1.71 kg), and sodium acetate (1.64 kg) were added into a reactor, heated to 65°C to 70°C and reacted completely. A resulting reaction system was cooled, and MTBE (13.0 kg) was added thereto. A resulting mixture was filtered to obtain a filtrate, and the filtrate was adjusted to have a pH of 4 to 8 by adding a potassium carbonate aqueous solution into the filtrate. The resulting adjusted filtrate was subjected to liquid separation to collect an organic phase, and the organic phase was subjected to concentration, recrystallization, filtration and drying to obtain product A (2.84 kg, with a yield of 70% and a purity of 99.73% (see FIGs. 12A and 12B)); where
      step (1) was conducted according to the following reaction equation: 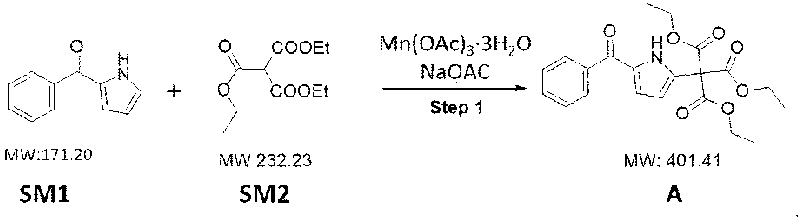
   (2) 1,2-dichloroethane (59.0 kg), the product A (2.82 kg), potassium carbonate (10.0 kg), and TBAB (2.28 kg) were added into a reactor, and heated to a temperature of 75°C to 80°C and reacted completely to obtain a mixed solution containing product B. The mixed solution containing the product B was filtered, a resulting filtrate was transferred to the reactor and subjected to concentration therein. After that, a THF solution (16.02 kg) and a sodium hydroxide solution (8.60 kg) were added into the reactor in sequence, and a resulting system was heated to a temperature of 50°C to 60°C and reacted completely. A resulting product was subjected to liquid separation, and a resulting organic phase was collected and adjusted to have a pH of 3 by adding a solution of HCl in THF (4.15 kg) thereto. A resulting mixture was concentrated to obtain a concentrate, and the MTBE (20.10 kg) was added into the concentrate. A resulting product was washed with water and then subjected to liquid separation, and a resulting organic phase was collected and subjected to decolorization and recrystallization, filtration and drying in sequence to obtain product C (1.06 kg, with a yield of 56% and a purity of 94.97% (see FIGs. 13A and 13B)), i.e., the ketorolac; where
      step (2) was conducted according to the following reaction equation: 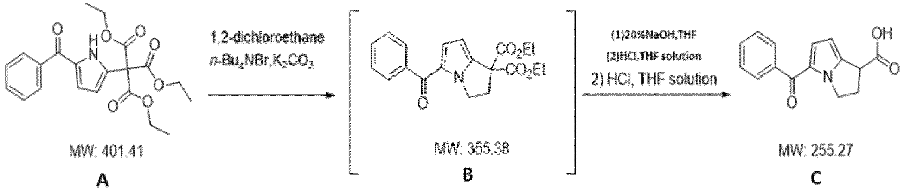
Step 2, Resolution of the (R)-ketorolac:
   The product C (1.06 kg) and isopropyl alcohol (4.64 kg) were added into a reactor, and heated at a temperature of 50°C to 60°C, such that the product C was dissolved to obtain a system. A chiral amine (1.50 kg) and a seed crystal (0.018 kg) were added into the system, and stirred to be uniform, and ethyl acetate (5.42 kg) was then added thereto dropwise. A resulting system was subjected to cooling crystallization and then filtered to obtain solid 1 and a filtrate. The solid 1 was acidified with hydrochloric acid (6.20 kg), and a resulting acidified system was filtered to obtain product FP (0.452 kg, with a yield of 42%, a purity of 99.50% (see FIGs. 14A and 14B), and a chiral purity of 99.58% (see FIGs. 15A and 15B)), i.e., the (R)-ketorolac; where
   Step 2 was conducted according to the following reaction equation:

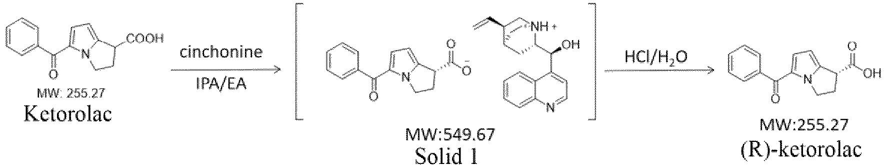

**Use Example:** Use of (R)-ketorolac in preparation of a drugs for treating colon cancer or breast cancer
1. Therapeutic effect of (R)-ketorolac combined with gemcitabine on colon cancer model Colon-26

The colon cancer tumor model was established by using adult male BALB/C mice which were subcutaneously inoculated 106 well-growing Colon-26 tumor cells. Starting from the 7th day after tumor inoculation, the two largest diameters of the tumors were measured with vernier calipers twice a week, and the tumor size was expressed as area (square millimeters). After the tumors grew to a certain size, the mice were randomly divided into 4 groups, including a control group (n=5) and 3 treatment groups: the (R)-ketorolac monotherapy group (n=5) was administered by gavage, 0.1 mg/d, for a total of 12 d; the gemcitabine monotherapy group (n=4) was administered intraperitoneally, 2 mg/1 time every 3 d, for a total of 4 times; and the combined therapy group (n=5) adopted the two drugs in combination, and the dosage and frequency of administration were the same as those in the monotherapy group.

After starting the treatment, the tumors were measured twice a week, and a tumor growth curve was made based on average tumor area ± SEM. Form the growth curve as shown in FIG. 16, it can be seen that, although alone administration of (R)-ketorolac has no effect on colon cancer tumor growth, the combination with gemcitabine could significantly enhance the inhibitory effect on tumor growth (**p*=0.021).

2. Therapeutic effect of (R)-ketorolac combined with cyclophosphamide on colon cancer model Colon-26

The colon cancer tumor model was established as described in the above. When the tumors grew to a certain size, the mice were randomly divided into 3 groups, including 1 control group (n=6) and 2 treatment groups (a cyclophosphamide monotherapy group and a combined therapy group). The cyclophosphamide monotherapy group (n=6) was administered by intraperitoneal injection, once every 6 d, 3 mg each injection, for a total of 3 injections. For the combined therapy group (n=7), the dosage and frequency of administration of cyclophosphamide were the same as those in the monotherapy group, and on this basis (R)-ketorolac was administered intragastrically, once a day, 0.1 mg each time, for a total of 18 times.

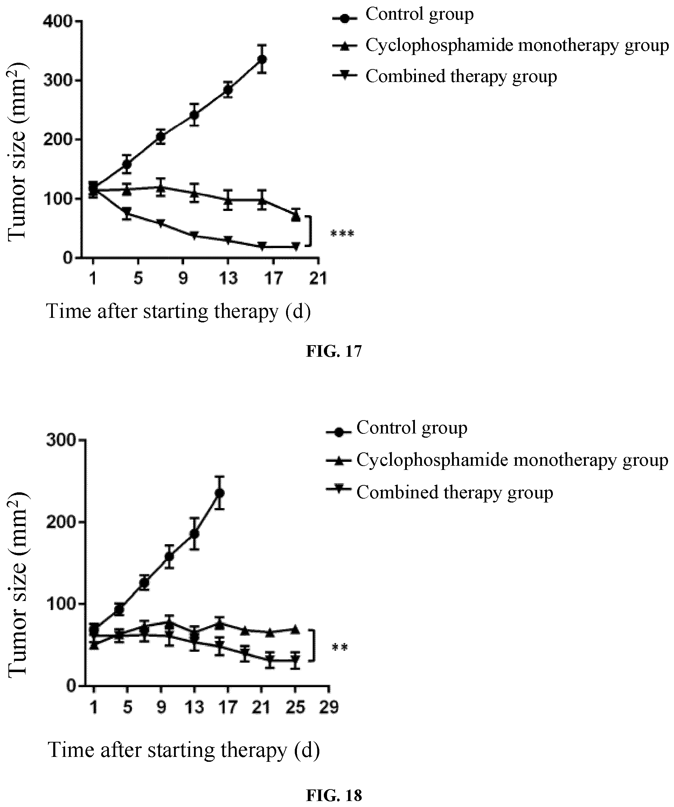

After starting the treatment, the tumors were measured twice a week, and a tumor growth curve was made based on average tumor area ± SEM. From the growth curve as shown in FIG 17, it can be seen that the combination administration of (R)-ketorolac and cyclophosphamide could significantly enhance the inhibitory effect on tumor growth (****p*=0.0002). More importantly, 4 out of 7 mice in the combined therapy group are completely cured (showing a cure rate of 57.1%), while only 1 out of 6 mice in the cyclophosphamide group are cured (showing a cure rate of 16.7%). This proves that the cure rate of the combined therapy group is 3.4 times that of the cyclophosphamide monotherapy group.

3. Therapeutic effect of (R)-ketorolac combined with cyclophosphamide on breast cancer model 4T1

The breast cancer tumor model was established by using adult female BALB/C mice which was subcutaneously inoculated 5×10⁵ well-growing 4T1 tumor cells. Starting from the 7th day after tumor inoculation, the two largest diameters of the tumors were measured with vernier calipers twice a week, and the tumor size was expressed as area (square millimeters). When the tumors grew to a certain size, the mice were randomly divided into 3 groups, including 1 control group (n=5) and 2 treatment groups (a cyclophosphamide monotherapy group and a combined therapy group). The cyclophosphamide monotherapy group (n=6) was administered by intraperitoneal injection, once every 6 d, 3 mg each injection, for a total of 4 injections. For the combined therapy group (n=6), the dosage and frequency of administration of cyclophosphamide were the same as those in the monotherapy group, and on this basis (R)-ketorolac was administered intragastrically, once a day, 0.1 mg each time, for a total of 24 times.

After starting the treatment, the tumors were measured twice a week, and a tumor growth curve was made based on average tumor area ± SEM. From the growth curve as shown in FIG 18, it can be seen that the combination administration of (R)-ketorolac and cyclophosphamide could significantly enhance the inhibitory effect of cyclophosphamide on the growth of 4T1 breast cancer tumors (***p*=0.0075).

Finally, it should be noted that the foregoing embodiments are only intended to describe, rather than to limit the technical solutions of the present disclosure. Although the present disclosure is described in detail with reference to the embodiments, a person of ordinary skill in the art should understand that modifications or equivalent replacements may be made to the technical solutions of the present disclosure without departing from the concept and scope of the technical solutions of the present disclosure.

## Claims

1. A method for preparing (R)-ketorolac, comprising the following steps:
step 1, preparing ketorolac; and
step 2, subjecting the ketorolac to resolution to obtain the (R)-ketorolac, the resolution being selected from the group consisting of chiral amine resolution and enzyme resolution;
wherein the chiral amine resolution comprises steps of:
adding the ketorolac and isopropyl alcohol into a first reactor, heating to dissolve the ketorolac, adding a chiral amine and a seed crystal thereto, stirring to be uniform, adding ethyl acetate dropwise thereto, subjecting a resulting mixture to cooling crystallization and filtration in sequence to obtain solid 1 and a filtrate, acidifying the solid 1 with hydrochloric acid to obtain an acidified product, and filtering the acidified product to obtain product FP, i.e., the (R)-ketorolac;
wherein the chiral amine resolution is conducted according to the following reaction equation: 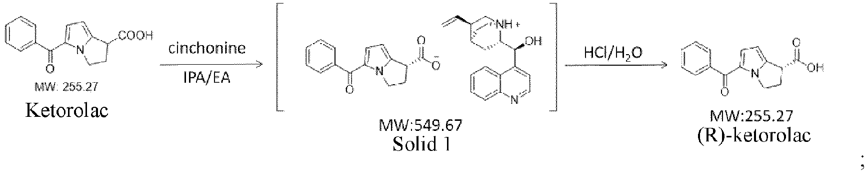 and
the enzyme resolution comprises steps of:
(i) adding the ketorolac and methanol into a second reactor, cooling to a temperature of -5°C to 5°C, adding SOCl₂ thereto, heating a resulting system to a temperature of 40°C to 50°C and reacting completely to obtain a reactant, subjecting the reactant to concentration, then crystallization by adding water, filtration, and drying in sequence to obtain product D, i.e., ketorolac methyl ester;
wherein step (i) is conducted according to the following reaction equation: 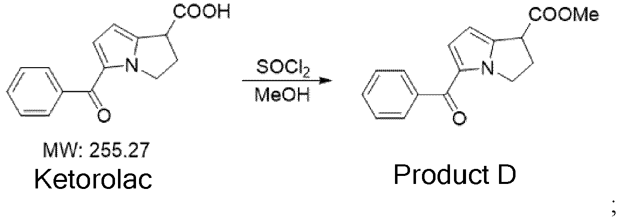 and
(ii) adding the product D, tert-butyl alcohol (TBA), a buffer solution, and an enzyme into a third reactor in sequence, and reacting while monitoring a chiral purity; after the reacting is completed, filtering a resulting reaction product to obtain a filtrate, washing with methyl tert-butyl ether (MTBE) and water, and subjecting a resulting washed system to liquid separation to obtain an organic phase, subjecting the organic phase to concentration and then further liquid separation to obtain a further organic phase, collecting the further organic phase and adding MTBE, cooling to a temperature of -5°C to 5°C, adding isopropylamine thereto and stirring, filtering a resulting system to obtain a solid; adding the solid into the reactor and then acidifying the solid with water and 4 mol/L hydrochloric acid aqueous solution to obtain an acidified product, and filtering the acidified product to obtain product FP, i.e., the (R)-ketorolac;
wherein step (ii) is conducted according to the following reaction equation:

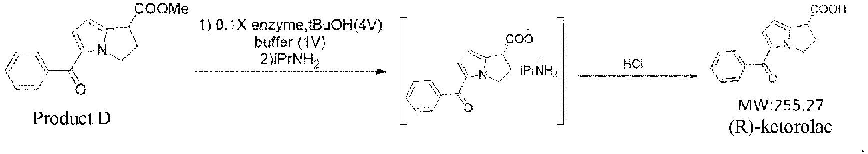

2. The method according to claim 1, wherein preparing ketorolac in step 1 comprises the following steps:
(1) adding glacial acetic acid, manganese acetate dihydrate, triethyl methanetricarboxylate (SM2), 2-benzoylpyrrole (SM1), and sodium acetate into a fourth reactor, heating and reacting completely to obtain a reaction system, cooling the reaction system, adding MTBE thereto, and filtering a resulting system to obtain a filtrate; adjusting the filtrate to have a pH value of 4 to 8 by adding a potassium carbonate aqueous solution, then subjecting the filtrate to liquid separation to collect an organic phase, subjecting the organic phase to concentration, recrystallization, filtration, and drying in sequence to obtain product A, i.e., triethyl (5-benzoyl-1H-pyrrol-2-yl)methanetricarboxylate;
wherein step (1) is conducted according to the following reaction equation: 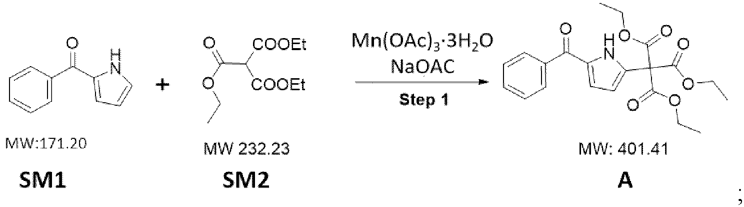 and
(2) adding 1,2-dichloroethane, the product A, potassium carbonate, and tetrabutylammonium bromide (TBAB) into a fifth reactor, and subjecting a resulting system to first heating, and reacting completely to obtain a mixed solution containing product B, i.e., diethyl 5-benzoyl-2,3-dihydro-1H-pyrrolizine-1,1-dicarboxylate; filtering the mixed solution containing the product B to obtain a filtrate, introducing the filtrate into a sixth reactor and conducting concentration, then adding a tetrahydrofuran (THF) solution and a sodium hydroxide solution into the sixth reactor in sequence, subjecting a resulting system to second heating, and reacting completely to obtain a product, subjecting the product to liquid separation to obtain an organic phase, collecting the organic phase and adjusting the organic phase to have a pH value of 3 by adding a solution of HCl in THF solution of HCl to obtain a mixture solution, subjecting the mixture solution to concentration to obtain a concentrate, adding MTBE into the concentrate, and washing with water, subjecting a resulting washed product to liquid separation to obtain an organic phase, collecting the organic phase, subjecting the organic phase to decolorization, recrystallization, filtration, and drying in sequence to obtain product C, i.e., the ketorolac;
wherein step (2) is conducted according to the following reaction equation:

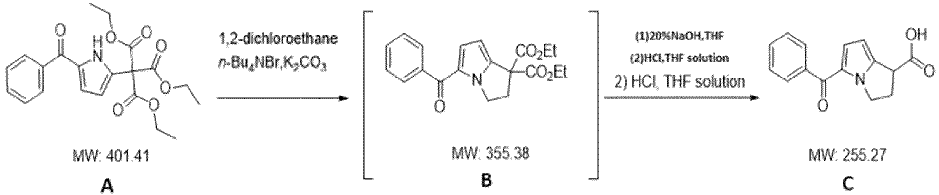

3. The method according to claim 1, wherein in the chiral amine resolution, the isopropyl alcohol is added in an amount of a mass ratio of the isopropyl alcohol to the ketorolac being 4.2:1 to 8.0:1.

4. The method according to claim 1, wherein in the chiral amine resolution, the heating is conducted at a temperature of 50°C to 60°C.

5. The method according to claim 1, wherein in the chiral amine resolution, the chiral amine is one selected from the group consisting of dehydroabietylamine (DHAA), (s)-1-phenylethylamine, (1S,2S)-(+)-1,2-diaminocyclohexane, L-(-)-epinephrine, (R)-(+)-1-(1-naphthyl)ethylamine, and cinchonine.

6. The method according to claim 1, wherein in the chiral amine resolution, a molar ratio of the ketorolac to the chiral amine is in a range of 1:0.82 to 1:1.73.

7. The method according to claim 1, wherein in the chiral amine resolution, a molar ratio of the ketorolac to the seed crystal is in a range of 1:0.0005 to 1:0.05.

8. The method according to claim 1, wherein in the chiral amine resolution, the chiral amine is added by batch addition; and the batch addition comprises steps of:
firstly adding the chiral amine at a molar ratio of the chiral amine to the ketorolac being 0.52:1 to 0.86:1, and dissolving solid by stirring,
secondly adding the chiral amine at a molar ratio of the chiral amine to the ketorolac being 0.04: 1 to 0.17 :1, and dissolving the solid by stirring,
thirdly adding the chiral amine at a molar ratio of the chiral amine to the ketorolac being 0.04:1 to 0.17 : 1, stirring for 1 h,
fourthly adding the chiral amine at a molar ratio of the chiral amine to the ketorolac being 0.04: 1 to 0.17 :1, stirring for 1.5 h, and
finally adding the chiral amine at a molar ratio of the chiral amine to the ketorolac being 0.17:1 to 0.35 : 1.

9. The method according to claim 8, wherein the seed crystal is added after secondly adding the chiral amine.

10. The method according to claim 1, wherein in the chiral amine resolution, the ethyl acetate is added in an amount of a mass ratio of the ethyl acetate to the ketorolac being 5: 1, and the method further comprises stirring at a temperature of 50°C to 60°C for 6 h to 12 h after adding the ethyl acetate dropwise is completed.

11. The method according to claim 1, wherein in the chiral amine resolution, the cooling crystallization is conducted by cooling to a temperature of 15°C.

12. The method according to claim 1, wherein in the enzyme resolution, the enzyme is Novozym 435, and the enzyme is added in an amount of a mass ratio of the enzyme to the product D being 10:1.

13. The method according to claim 2, wherein in step (1), a molar ratio of the 2-benzoylpyrrole, the triethyl methanetricarboxylate, the manganese acetate dihydrate, the sodium acetate, and the glacial acetic acid is in a range of 0.99-1.01 : 1.09-1.12 : 1.9-2.1 : 2.8-3.2 : 28-32.

14. The method according to claim 2, wherein in step (1), a mass ratio of the MTBE to the 2-benzoylpyrrole is 18:1.

15. The method according to claim 2, wherein in step (2), a molar ratio of the 1,2-dichloroethane, the product A, the potassium carbonate, and the TBAB is in a range of 85-95 : 0.99-1.01 : 8.7-11.6 : 0.75-1.25.

16. The method according to claim 2, wherein in step (2), a mass ratio of the THF solution to the product A is 8:1, and a mass ratio of the sodium hydroxide solution to the product A is 4:1.

17. The method according to claim 16, wherein the sodium hydroxide solution has a mass percentage of 20%.

18. The method according to claim 2, wherein in step (2), the solution of HCl in THF has a mass percentage of 11%, and the solution of HCl in THF is added in an amount of a mass ratio of the solution of HCl in THF to the product A being 1.45:1.

19. The method according to claim 2, wherein in step (2), a mass ratio of the MTBE to the product A is 7:1.

20. The method according to claim 2, wherein in step (2), the recrystallization is performed by: concentrating a decolorized organic phase to obtain a concentrated organic phase, adding n-heptane into the concentrated organic phase to obtain a mixture, heating the mixture to a temperature of 40°C and dissolving a resulting solid while stirring to obtain a mixed solution, slowly cooling the mixed solution to a temperature of -10°C so that the resulting solid is crystallized, and subjecting a resulting product to filtration and vacuum drying in sequence to obtain the product C.

21. (R)-ketorolac prepared by the method according to any one of claims 1 to 20 for use in preparation of a drug for treating a cancer.

22. The (R)-ketorolac for use in preparation of the drug for treating the cancer according to claim 21, wherein the cancer is at least one selected from the group consisting of colon cancer and breast cancer.

23. The (R)-ketorolac for use in preparation of the drug for treating the cancer according to claim 21, wherein the drug for treating the cancer comprises the (R)-ketorolac and a chemotherapeutic drug; and the chemotherapeutic drug comprises one selected from the group consisting of gemcitabine and cyclophosphamide.
